# EUROPEAN PATENT SPECIFICATION

(11) **EP 4 346 573 B1**
(45) Date of publication and mention of the grant of the patent: **11.03.2026**
(21) Application number: 22814595.9
(22) Date of filing: 18.05.2022
(51) Int. Cl.: A61B 5/024, A61B 5/288, A61B 5/344, A61B 5/251, G01N 27/327, A61B 5/0205, A61B 5/1482, C12Q 1/00, A61B 5/0245, A61B 5/00, A61B 5/145, A61B 5/1486

(54) **FOETAL MONITORING SYSTEM**
FÖTUSÜBERWACHUNGSSYSTEM
SYSTÈME DE SURVEILLANCE FOETALE

(30) Priority: 31.05.2021 AU 2021901626; 11.10.2021 AU 2021903268
(43) Date of publication of application: 10.04.2024
(73) Proprietor: Vitaltrace Pty Ltd, Wembley WA 6014 (AU)
(72) Inventor: KAUSHIK, Arjun Siddharth, City Beach, Western Australia 6015 (AU); CHALLENOR, Michael Tom, Maylands, Western Australia 6051 (AU); ATKINSON, Robert David John, Bentley, Western Australia 6102 (AU)
(74) Representative: Potter Clarkson
(86) International application number: PCT/AU2022/050478
(87) International publication number: WO 2022/251897

(56) References cited:
- EP-B1- 1 673 011
- WO-A1-2015/185745
- WO-A1-2020/069572
- WO-A1-2021/076940
- WO-A1-2023/150840
- US-A- 4 658 825
- US-A1- 2008 319 294
- SHULGIN VYACHESLAV ET AL: "Spatio-temporal signal processing for fetus and mother state monitoring during pregnancy", 2018 IEEE 9TH INTERNATIONAL CONFERENCE ON DEPENDABLE SYSTEMS, SERVICES AND TECHNOLOGIES (DESSERT), IEEE, 24 May 2018 (2018-05-24), pages 641 - 644, XP033374715, DOI: 10.1109/DESSERT.2018.8409210
- KEENAN EMERSON ET AL: "Personalized Anatomic Modeling for Noninvasive Fetal ECG: Methodology and Applications", IEEE TRANSACTIONS ON INSTRUMENTATION AND MEASUREMENT, IEEE, USA, vol. 70, 29 March 2021 (2021-03-29), pages 1 - 12, XP011849525, ISSN: 0018-9456, [retrieved on 20210414], DOI: 10.1109/TIM.2021.3069028

## Description

### TECHNICAL FIELD

The present invention relates to a foetal monitoring device for simultaneously monitoring an analyte in a foetus and the heart rate of the foetus.

### BACKGROUND ART

During labour and delivery, there is a risk of damage to the foetal brain, neurological system and other end organs as a result of hypoxia, otherwise known as foetal asphyxia. Foetal asphyxia can cause long term, debilitating sequelae for the baby and their family.

Currently, clinicians monitor uterine contractions and foetal heart rate (FHR) using a cardiotocograph (CTG) wherein a change in signal patterns may indicate foetal asphyxia. However, the change in signal patterns are typically only assessed subjectively, which has led to a high number of false positives. As a consequence, many caesarean sections are performed based upon fallacious indication of foetal distress as a defensive measure to reduce the risk of intrapartum foetal asphyxia. Due to the large number of false positives, the rate of non-elective caesarean sections has increased. However, caesarean sections come with a number of problems. For example, caesarean sections are associated with increased morbidity for the mother, longer recovery times, and higher rates of post-partum infections (to name a few). Furthermore, caesarean sections are higher in cost than vaginal deliveries.

Previous systems have attempted to use various continuous monitoring technologies, such as in US 2017/0112428 which used a micro-dialysis technique to sample extracellular fluid to determine analyte concentrations like lactate. However, this still results in significant lag between the result and sampling due to the micro-dialysis process.

Another method to monitor foetal health during delivery is provided in WO 03/088837, which combined a temperature probe with an ECG. However, whilst this allowed for continuous monitoring of temperature, this parameter is prone to interference given the close proximity of the mother and there is a lower correlation between temperature and foetal asphyxia than some other parameters. WO 2020/069572 discusses a device, system, and method for monitoring an analyte concentration in a foetus. In particular, the document relates to a device, system, and method for monitoring a lactate concentration in foetal tissue.

It would be advantageous if at least an embodiment of the present invention provided a method to monitor a parameter associated with foetal asphyxia continuously and in real-time, or at least provide an alternative to conventional devices and methods.

There is a need to monitor a parameter associated with foetal asphyxia continuously and in real-time, or at least provide an alternative or compliment to conventional devices and methods. The present invention seeks to provide an improved or alternative method for monitoring foetal asphyxia continuously and in real-time.

The previous discussion of the background art is intended to facilitate an understanding of the present invention only. The discussion is not an acknowledgement or admission that any of the material referred to is or was part of the common general knowledge as at the priority date of the application.

### SUMMARY OF INVENTION

The present invention provides a foetal monitoring device as indicated in claim 1.

Preferably the analyte is a lactate analyte or an oxygen analyte. If the analyte is a lactate analyte, preferably the reactive substance of the first electrode is an immobilised enzyme. If the analyte is an oxygen analyte, preferably the reactive substance of the first electrode is platinum or another noble metal.

Preferably the second electrode in the form of a biosensor reference electrode (BRE) provides a stable reference voltage to assist with monitoring the analyte concentration of the foetus.

Preferably the third electrode in the form of an electrocardiogram working electrode (EWE) monitors the foetal heart rate of the foetus.

Preferably the fourth electrode in the form of an electrocardiogram reference electrode (ERE) provides a reference measurement in contact with the amniotic fluid.

In the invention, the second electrode (BRE) is also the third electrode (EWE) to form a combined BRE/EWE electrode.

In one embodiment, the second electrode (BRE) is also the biosensor counter electrode (BCE). Alternatively, the third electrode (EWE) is also the biosensor counter electrode (BCE), or the combined BRE/EWE electrode is the BCE.

Another embodiment further provides a system for simultaneously monitoring a concentration of an analyte in a foetus and a heart rate of the foetus, the system comprising:
A. a foetal monitoring device comprising five electrodes, the device comprising:
   a) two electrodes forming a biosensor wherein:
      (i) the first electrode is a biosensor working electrode (BWE), comprising a reactive substance, for monitoring the analyte concentration of the foetus,
      (ii) the second electrode is a biosensor reference electrode (BRE) for monitoring the analyte concentration of the foetus,
   b) three electrodes forming an electrocardiogram (ECG) wherein:
      (iii) the third electrode is an electrocardiogram working electrode (EWE),
      (iv) the fourth electrode is an electrocardiogram reference electrode (ERE) for monitoring the heart rate of the foetus,
      (v) the fifth electrode is an electrocardiogram ground electrode (EGE), connected externally to the mother, for monitoring the heart rate of the foetus
   c) a device body for supporting the five electrodes, the device body being configured to contact a surface area of the foetal tissue;
B. an analysis component in electronic communication with the biosensor and/or the ECG, the analysis component being configured to determine the concentration of the analyte and/or the foetal heart rate using the detected electronic signal of the biosensor and/or ECG

wherein the first electrode is not in physical contact with, or is electrically isolated from, the second electrode;
wherein the third electrode is not in physical contact with the first electrode;
wherein the fourth electrode is not in physical contact with the first, second, or third electrode;
wherein the fifth electrode is not in physical contact with the first, second, third, or fourth electrode;
wherein the foetal monitoring device is configured such that:
   the first, second and third electrodes form one or more protrusions that can be at least partially inserted into the foetal tissue so that (i) the analyte in the foetal tissue electrochemically reacts with the reactive substance of the first electrode (BWE) and in response to the electrochemical reaction the first electrode (BWE) of the biosensor detects an electronic signal, wherein a parameter of the electronic signal is indicative of the concentration of the analyte or a rate of change of concentration of the analyte; (ii) the third electrode (EWE) of the ECG detects the electrical activity of the foetal heart; and (iii) when the device body contacts the surface area of the foetal tissue, the device body can be anchored to the foetal tissue using the first, second, and third electrodes as an anchor to secure the device body against the contacted surface area of the foetal tissue.

### BRIEF DESCRIPTION OF THE DRAWINGS

Further features of the present invention are more fully described in the following description of several non-limiting embodiments thereof. This description is included solely for the purposes of exemplifying the present invention. It should not be understood as a restriction on the broad summary, disclosure or description of the invention as set out above. The description will be made with reference to the accompanying drawings in which:
Figure 1 is a schematic representation of a system for simultaneously monitoring a concentration of an analyte in a foetus and a heart rate of the foetus in accordance with an embodiment of the present invention.
Figure 2 is a schematic representation of a system for simultaneously monitoring a concentration of an analyte in a foetus and a heart rate of the foetus in accordance with an embodiment of the present invention.
Figure 3 is a schematic representation of a device of the present invention, where the BWE is a needle-shaped protrusion and a spiral protrusion forms both the BRE and EWE.
Figure 4 is a schematic representation of an (unclaimed) embodiment, where the BWE and BRE are located in close proximity in a needle-shaped protrusion and the EWE is in the form of a spiral protrusion.
Figure 5 is a schematic representation of an (unclaimed) embodiment, where the BWE and BRE are located in close proximity in a needle-shaped protrusion and the EWE is combined with the BCE and is in the form of a spiral protrusion.
Figure 6 is an expanded schematic of a device of the present invention, where the BWE is a needle-shaped protrusion and a spiral protrusion forms both the BRE and EWE.
Figure 7 is an expanded schematic of an (unclaimed) embodiment, where the BWE and BRE are located in close proximity in a needle-shaped protrusion and the EWE is in the form of a spiral protrusion.
Figure 8 is a schematic of an (unclaimed) embodiment, where the BWE and BRE are located in close proximity in a needle-shaped protrusion and the EWE is combined with the BCE and is in the form of a spiral protrusion.
Figure 9 is a plot of current (µA) recorded by a device of the present invention, where the BWE is a needle-shaped protrusion and a spiral protrusion forms both the BRE and EWE, and venous blood lactate (mmol/L) of a newly born sheep recorded by an external blood gas device.
Figure 10 is a plot of current (µA) recorded by an (unclaimed) embodiment where the BWE and BRE are located in close proximity in a needle-shaped protrusion and the EWE is in the form of a spiral protrusion, and venous blood lactate (mmol/L) of a newly born sheep recorded by an external blood gas device.

### DESCRIPTION OF INVENTION

### Detailed Description of the Invention

False positive indications of foetal distress as a result of conventional methods of monitoring the well-being of a foetus through heart rate monitoring alone may result in an increase in caesarean sections to reduce the time of the delivery. One method to monitor the well-being of a foetus relates to collecting a sample of foetal blood to determine foetal blood lactate levels. Lactate is a metabolite produced during glucose metabolism in the presence of asphyxia. The sample of foetal blood is typically collected from the foetal scalp or umbilical arteries at the delivery. The sample is then analysed for the concentration of lactate which is produced when the foetus does not have enough oxygen and undergoes anaerobic respiration. However, this method has a number of disadvantages. For example, the method is relatively invasive for the foetus and the mother. Due to its complexity, it is difficult to perform which results in a median time of approximately 20 minutes for results. Furthermore, complications such as hematoma, bleeding from the scalp, and death can occur as a result of sample collection.

The present invention aims to reduce the complexity and invasiveness of monitoring for foetal distress. Furthermore, the present invention enables the monitoring of a selected analyte, such as lactate or oxygen, continuously and in real-time, and the simultaneous monitoring of the foetal heart rate. As such, a doctor or midwife can act immediately once oxygen levels drop below a certain threshold, lactate levels exceed a predetermined threshold, or the heart rate changes.

### Monitoring Device

The present invention provides a foetal monitoring device comprising five electrodes for simultaneously monitoring a concentration of an analyte in a foetus and a heart rate of the foetus, the device comprising:
a) two electrodes forming a biosensor for monitoring a concentration of an analyte in the foetus wherein:
   (i) the first electrode is a biosensor working electrode (BWE), comprising a reactive substance, for monitoring the analyte concentration of the foetus,
   (ii) the second electrode is a biosensor reference electrode (BRE) for monitoring the analyte concentration of the foetus,
b) three electrodes forming an electrocardiogram (ECG) for monitoring the heart rate of the foetus wherein:
   (iii) the third electrode is an electrocardiogram working electrode (EWE),
   (iv) the fourth electrode is an electrocardiogram reference electrode (ERE) for monitoring the heart rate of the foetus,
   (v) the fifth electrode is an electrocardiogram ground electrode (EGE), connected externally to the mother, for monitoring the heart rate of the foetus,
c) a device body for supporting the five electrodes, the device body being configured to contact a surface area of foetal tissue

wherein the first electrode is not in physical contact with, or is electrically isolated from, the second electrode;
wherein the third electrode is not in physical contact with the first electrode; wherein the second electrode (BRE) is also the third electrode (EWE) to form a combined BRE/EWE electrode; Z
wherein the fourth electrode is not in physical contact with the first, second, or third electrode;
wherein the fifth electrode is not in physical contact with the first, second, third, or fourth electrode;
wherein the foetal monitoring device is configured such that:
   the first, second and third electrodes form one or more protrusions that can be at least partially inserted into the foetal tissue so that (i) the analyte in the foetal tissue electrochemically reacts with the reactive substance of the first electrode (BWE) and in response to the electrochemical reaction the first electrode (BWE) of the biosensor detects an electronic signal, wherein a parameter of the electronic signal is indicative of the concentration of the analyte or a rate of change of concentration of the analyte; (ii) the third electrode (EWE) of the ECG detects the electrical activity of the foetal heart; and (iii) when the device body contacts the surface area of the foetal tissue, the device body can be anchored to the foetal tissue using the first, second, and third electrodes as an anchor to secure the device body against the contacted surface area of the foetal tissue.

In one embodiment, the second electrode (BRE) is the biosensor counter electrode (BCE). Alternatively, the third electrode (EWE) is the biosensor counter electrode (BCE).

In the invention, the second electrode (BRE) is also the third electrode (EWE).

Preferably the first electrode (BWE) is in the form of a needle, rod or wire. Preferably the second electrode (BRE) is in the form of a spiral or hook, or in the form of a micro-coil or paste placed around the biosensor working electrode (BWE). Together, the needle and the spiral form protrusions that can be at least partially inserted into the foetal tissue and act as an anchor for the foetal monitoring device.

Embodiments of the present invention enable the real-time monitoring of a concentration of a selected analyte in the foetus. In this way, analytes such as foetal oxygen, oxygen metabolism end products and metabolites of the foetus can be examined in real-time which may provide an indication of the well-being of the foetus during labour and delivery. Furthermore, the process of electrochemically detecting the concentration of the analyte in the foetus may be less invasive for the foetus, as it may not be necessary to take a sample of tissue or blood from the foetus.

Preferably the analyte is a lactate analyte or an oxygen analyte. If the analyte is a lactate analyte, preferably the reactive substance of the first electrode is an immobilised enzyme. If the analyte is an oxygen analyte, preferably the reactive substance of the first electrode is platinum or other noble metals such as ruthenium, rhodium, palladium, osmium, iridium, gold or silver.

Furthermore, embodiments of the present invention enable the real-time monitoring of the heart rate of the foetus. In this way, foetal heart rate can be examined in real-time which may provide an indication of the well-being of the foetus during labour and delivery.

The present invention provides for the consolidation of the biosensor and the ECG apparatus, whereby five electrodes function as two sets of electrodes. In the present invention, one electrode pair is an electrochemical biosensor electrode set consisting of a BWE and a BRE (the first and second electrodes), and the second electrode set is an ECG apparatus consisting of an EWE, an ERE, and an EGE (the third, fourth, and fifth electrodes).

In one embodiment of the present device, the biosensor reference electrode (BRE) takes the form of a spiral or hook and the biosensor working electrode (BWE) is in the form of a needle-shaped protrusion. This embodiment facilitates greater BRE and BWE contact with foetal tissue, as both protrusions at least partially enter the foetal tissue and holds the device in place as an anchor.

The biosensor reference electrode (BRE) in the form of a spiral is combined in function with the ECG apparatus working electrode (EWE). The EWE may be made from a conductive biologically compatible non-toxic material such as: a metal (e.g. stainless steel, silver, titanium) or a biological or synthetic non-metallic conductive material. The EWE is then converted into a BRE/EWE by coating the conductive metal spiral with a reference layer of silver/silver chloride. The silver/silver chloride reference layer allows for the creation of a stable reference voltage for the biosensor. The silver/silver chloride reference layer may then be coated with an additional protective layer to protect the silver from mechanical damage during insertion of the spiral into the foetal tissue. Preferably the protective layer is polyurethane or perfluoro-3,6-dioxa-4-methyl-7-octenesulfonic acid-tetrafluoroethylene copolymer (Nafion^{™}). An example of a BRE/EWE may be made by coating a titanium spiral with silver chloride, and then covering the silver chloride layer with polyurethane. The combination of the BRE with the EWE allows for a reduction in the number of cables (four wires gets reduced to three) and also a reduced number of solder joints within the sensor module. This embodiment facilitates greater BRE/EWE contact with the foetal tissue as the BRE/EWE is preferably in the form of a screw protrusion that at least partially enters the foetal tissue and holds the device in place as an anchor.

It is preferred that the central biosensor working electrode (BWE) in the form of a needle, rod or wire, protrudes beyond the spiral acting as the EWE/BRE. The extension of the needle beyond the spiral allows for superior penetration and creates a more reliable hold on the foetal tissue. Alternatively it is preferred that the central biosensor working electrode (BWE) in the form of a needle, rod or wire does not extend beyond the spiral acting as the EWE/ BRE. This enables the spiral to pierce the skin first, providing a firm hold prior to insertion of the BWE.

In another embodiment, the biosensor reference electrode (BRE) takes the form of a micro-coil or metal layer and is placed around the biosensor working electrode (BWE), from which it is electrically insulated. In this embodiment, the BWE and the BRE are electrically separated from each other by an insulating layer. The insulating layer allows the biosensor reference electrode (BRE) and the biosensor working electrode (BWE) to be placed adjacent to each other without interacting electrically. The insulating layer may be made from dielectric ink or other electrical insulator material. In this embodiment, the ECG working electrode (EWE) takes the form of a spiral that at least partially enters the foetal tissue and holds the device in place as an anchor. In this embodiment, it is preferred that the central biosensor working electrode (BWE), protrudes beyond the spiral acting as the ECG working electrode (EWE). The extension of the needle beyond the spiral allows for superior penetration and creates a more reliable hold on the foetal tissue.

In the embodiments above, the ECG working electrode (EWE) may optionally act as the biosensor counter electrode (BCE).

The separation of the BCE and BRE improves the electronic signal by reducing or preventing the flow of current through the BRE. This improves the stability of the reference voltage on the BRE and directs all current through the larger BCE.

It is preferred that the BWE and BRE are spatially close to reduce voltage drop due to resistance between the two electrodes.

The foetal tissue into which the one or more protrusions that form an anchor are inserted may, for example, be located at a scalp of the foetus. However, it will be appreciated that the foetal tissue may be located at a different body part of the foetus, for example, if the foetus is in breech position.

### Biosensor Working Electrode

The biosensor working electrode (BWE) of the biosensor may be in the form of a needle. For example, an outer surface area of the needle may be coated with the reactive substance of the biosensor. Thus, by inserting the needle into the foetal tissue, the reactive substance can electrochemically react with the analyte in the tissue. In this example, the BWE may be configured to withstand forces when the needle is inserted into the biological tissue. Alternatively, the BWE may be in the form of a rod or a wire.

In one embodiment of the device, the BWE may be composed of a metal, coated with a mediator layer, reactive substance and protective layer, in that order. The metal must be conductive (to allow the flow of current) and a suitable electrode surface (for example to enable to detection of a lactate analyte, the electrode surface must be able to catalyse the reduction of H₂O₂; to enable to detection of an oxygen analyte, the electrode surface must be able to react with oxygen). In one embodiment of the device, the BWE is made from a metal such as platinum, gold or other rare earth metals. The platinum or other metal acts as the electrode surface and allows for sensing to take place. The metal electrode surface may then be coated with a mediator layer to catalyse the reaction and allow for detection of lactate at a lower voltage than possible without the mediator layer.

For example, if the analyte is a lactate analyte, a platinum BWE can sense lactate at around 0.5 V whereas a platinum BWE coated with a mediator layer of Prussian blue can sense lactate at around 0 V. This increases the accuracy and specificity of the biosensor by producing fewer undesirable side reactions with compounds such as acetaminophen or ascorbic acid. Exemplary mediator layer materials include: organic dyes based mediator layers such as methylene green, Meldola blue, tetrathiafulvalene or quinine groups; transition metal complexes such as cobalt phthalocyanine, hydroxymethyl ferrocene, osmium complexes with a variety of redox polymers; polymers such as poly(aniline)-poly(acrylate), poly(aniline)-poly(vinyl sulfonate), poly(pyrrole), poly(pyrrole)-poly(vinyl sulfonate) and poly(vinylpyrrolidone). Preferably the mediator layer is formed of Prussian blue (Fe^{III}₄[Fe^{II}(CN)₆]₃). The mediator layer may then be coated with the lactate oxidase reactive substance.

The reactive substance may then be coated with a protective layer to improve the mechanical stability of the BWE and reduce the migration of undesirable ions to the electrode surface. As well as mechanical protection and stability, the protective layer also preferably forms a negatively charged layer that prevents large negatively charged molecules from approaching the electrode.

For example, a lactate analyte can still reach the electrode surface as, despite its negative charge, it is small. Preferably the protective layer is a sulfonated tetrafluoroethylene-based fluoropolymer-copolymer such as perfluoro-3,6-dioxa-4-methyl-7-octenesulfonic acid-tetrafluoroethylene copolymer (Nafion^{™}). Preferably, if the analyte is a lactate analyte, the BWE is made of platinum, coated with a first layer of Prussian blue, a second layer of lactate oxidase, and a third layer of Nafion^{™}. This combination allows the BWE to sense lactate using a low applied voltage and prevent mass transport of undesirable ions to the surface of the electrode. If the analyte is oxygen, preferably the BWE is made of platinum or other noble metals.

In all embodiments, the ordering of the metal, the mediator layer, the reactive substance and the protective layer (in that order) is important. The metal provides the underlying electrode as well as the 'backbone', providing structural rigidity. For example, if the analyte is a lactate analyte, the mediator layer interacts with the electrode surface and must be in direct contact as the mediator layer (e.g. Prussian blue), in combination with the metal electrode (e.g. platinum), allows for reduction of H₂O₂. The reactive substance must be outside the mediator layer as it needs to interact with the solution surrounding the electrode (blood etc). If lactate is being detected, the H₂O₂ the reactive substance produces must pass to the metal/mediator layer to enable detection. The protective layer must be on the outside to improve mechanical stability of the layers and prevent undesirable ions from migrating to the surface.

For example, if the analyte is an oxygen analyte, the reactive layer may be composed primarily of platinum or another noble metal. Noble metals have the capacity to electrochemically reduced oxygen without the use of a mediator layer. In this case, the BWE may be composed of a core of platinum covered with the protective layer, such perfluoro-3,6-dioxa-4-methyl-7-octenesulfonic acid-tetrafluoroethylene copolymer (Nafion^{™}).

In an embodiment, the needle may comprise a hollow space wherein the BWE of the biosensor is at least partially located within the hollow space, and at least part of the inner surface of the needle may be coated with the reactive substance of the BWE of the biosensor. In one particular example, the needle comprises an opening to the hollow space within a side wall or at a tip of the needle to expose the BWE of the biosensor to an outer surface of the needle such that when the needle is inserted into the tissue, the reactive substance can electrochemically react with the analyte in the foetal tissue at the opening. The opening may, for example, be in the form of a slit or a cut out.

In a further example, the needle may comprise two sections: a hollow, tubular outer needle section and an inner tubular needle section comprising the BWE of the biosensor. The outer section of the needle may be moveable relative to the inner BWE section of the needle that is located within the hollow, tubular space of the outer needle. Thus, when the outer needle together with the inner needle has been inserted into the foetal tissue, the outer needle can be retracted while at least a portion of the inner BWE needle remains within the foetal tissue.

The BWE of the biosensor in the form of a needle may be made of any suitable material, including but not limited to platinum, stainless steel, biocompatible material, biological material, synthetic material and biodegradable material. The tissue penetrating portion of the wire comprising the needle may have a length between 0.5mm and 3mm, or 1mm and 2.5mm or 1.5mm and 2.5mm or 1mm and 2mm. The needle may have a length of approximately 5 mm, 10 mm, 15mm, or 20 mm. The wire comprising the needle may have a diameter of approximately 0.05 mm, 0.1 mm, 0.2 mm, 0.3 mm, 0.4, 0.5 mm or 0.6 mm.

The BWE of the biosensor in the form of a needle preferably enters the foetal tissue to a distance of approximately 0.5 mm, 1 mm, 1.5 mm, 2 mm, 2.5 mm or 3 mm.

### Biosensor Reference Electrode

In an embodiment, the biosensor reference electrode (BRE) may be in the form of a spiral or hook and the device may be configured such that the device body can be anchored to the foetal tissue by rotating the device body so that the spiral BRE enters the foetal tissue.

The BRE of the biosensor in the form of a spiral may be made of any suitable conductive material, including but not limited to: stainless steel, silver, titanium, biocompatible material, biological material, synthetic material and biodegradable material. The spiral should be sufficiently strong to maintain its shape when inserted into foetal tissue.

To allow the spiral to act as a BRE, the spiral is coated with a reference layer composed of any suitable redox couple that maintains a stable voltage. Silver/silver chloride is the most preferred material for a reference layer due to its stability and biological safety. The reference layer may be applied using electrodeposition, by application of adhesive ink (such as silver/silver chloride ink), or by electropolymerisation. By forming the spiral from a harder material such as stainless steel or titanium and coating the metal with a reference layer, the needle can be made tough enough to penetrate foetal tissue without deforming.

The BRE of the biosensor may further comprise a membrane layer outside the reference layer, which may be composed of cellulose, polyethylene, polyurethane or polypropylene or similar polymers. Its primary function is mechanical protection of the surface of the BRE.

The extended length of the spiral (if straightened out to a straight line) may have a length of approximately 1 cm, 2 cm, 3 cm, 4 cm, 5cm or 6 cm.

The wire comprising the spiral may have a diameter of approximately 0.2 mm, 0.3 mm, 0.4, 0.5 mm ,0.6 mm, or 0.7 mm.

The spiral may have a helical diameter of approximately 0.1 cm, 0.25 cm 0.5 cm, 1 cm or 2 cm.

The BRE of the biosensor in the form of a spiral preferably enters the foetal tissue to a distance of approximately 0.5 mm, 1 mm, 1.5 mm, 2 mm, 2.5 mm or 3 mm.

The BRE of the biosensor in the form of a spiral may have a distance of approximately 1 mm, 2 mm, 3, mm, 4 mm, 5 mm and 10 mm, 20 mm, 30 mm, 40 mm or 50 mm between the outer spiral and the needle BWE that is located within the centre of the spiral, or close to the centre of the spiral.

Alternatively, the biosensor reference electrode (BRE) may take the form of a micro-coil or metal layer and is placed around the base of the needle shaped biosensor working electrode (BWE), from which it is electrically insulated by the insulating layer. The insulating layer allows the biosensor reference electrode (BRE) and the biosensor working electrode (BWE) to be placed adjacent to each other without interacting electrically. The insulating layer may be made from dielectric ink or other dielectric material. In this embodiment, the BRE is still made from any suitable redox couple that maintains a stable voltage. Silver/silver chloride is the most preferred material for a BRE of this form. In this embodiment, the ECG working electrode (EWE) takes the form of a spiral that at least partially enters the foetal tissue and holds the device in place as an anchor.

### Biosensor Counter Electrode

The biosensor counter electrode (BCE) may be incorporated into the spiral EWE or the spiral BRE/EWE, or the BRE if it forms a microcoil around the BWE.

In all cases, it is preferred that the BCE has a greater surface area than the BWE. For example, the BWE may be 45% of the surface area of the BCE, 40%, 35%, 30%, 25%, 20% 15% or 10% of the surface area of the BCE. This increases accuracy of the biosensor as the rate limiting reaction occurs at the BWE rather than the BCE.

In embodiments where the BCE is part of the spiral EWE or the spiral BRE/EWE protrusion, the BWE has a surface area of between 90%-10% of that of the BCE. For example, the BWE may be 80% of the surface area of the BCE, 70%, 60%, 50%, 40%, 30%, 20% or 10% of the surface area of the BCE.

In embodiments where the BCE is part of the microcoil BRE, the BWE has a surface area of between 90%-10% of that of the BCE. For example, the BWE may be 80% of the surface area of the BCE, 70%, 60%, 50%, 40%, 30%, 20% or 10% of the surface area of the BCE.

### ECG Working Electrode

In an embodiment, the ECG working electrode (EWE) may be in the form of a spiral or hook and the device may be configured such that the device body can be anchored to the foetal tissue by rotating the device body so that the spiral EWE enters the foetal tissue.

The EWE of the ECG in the form of a spiral may be made of any suitable conductive material, including but not limited to: stainless steel, silver, titanium, biocompatible material, biological material, synthetic material and biodegradable material.

The extended length of the spiral (if straightened out to a straight line) may have a length of approximately 1 cm, 2 cm, 3 cm, 4 cm, 5cm or 6 cm.

The wire comprising the spiral may have a diameter of approximately 0.05 mm, 0.1 mm, 0.2 mm, 0.3 mm, 0.4, 0.5 mm or 0.6 mm.

The spiral may have a helical diameter of approximately 0.1 cm, 0.25 cm 0.5 cm, 1 cm or 2 cm.

The EWE of the ECG in the form of a spiral preferably enters the foetal tissue to a distance of approximately 0.5 mm, 1 mm, 1.5 mm, 2 mm, 2.5 mm or 3 mm.

The EWE in the form of a spiral may have a distance of approximately 1 mm, 2 mm, 3, mm, 4 mm, 5 mm and 10 mm, 20 mm, 30 mm, 40 mm or 50 mm between the outer spiral and the needle BWE that is located within the centre of the spiral, or close to the centre of the spiral.

### ECG Reference Electrode

The ECG Reference Electrode (ERE) is housed in the device body. The ERE may be composed of any substantially conductive material and may take a variety of forms. The ERE must form an electrical connection through the amniotic fluid of the vaginal canal with the EWE and so must protrude from the device body. The design of this is substantially similar to the design described in US Patent No: 3,827,428.

### ECG Ground Electrode

The EGE is attached to the mother, for example in the form of an adhesive patch, and measures the ECG of the mother so it can be deleted from the foetal signal to increase the specificity of the signal.

The EGE may take a variety of forms but all must be attached to the surface of the mother's body, preferably with a conductive gel. This mirrors current practice for external electrodes connected for ECG performed on adults. A person skilled in the art will appreciate that the ground electrode could be attached to any part of the mother's body and could form part of the external monitoring device.

### Device Body

The device body may further comprise a flexible material layer covering the surface area for contacting the foetal tissue.

The device may further comprise a guiding element that is configured such that the device body can be guided through the vaginal canal and the dilated cervix to position the device body against the foetal tissue. The guiding element may be attachable to the device body. For example, the guiding element may comprise a tube with a hollow portion and the device body may be positionable within the hollow portion of the guiding tube. Specifically, the guiding element may comprise a base tube and a sleeve that is movable relative to the base tube, wherein the guiding element may be configured such that a space for holding the device body is formed between the base tube and the sleeve.

The device body may comprise at least one recess for receiving at least a portion of the needle-shaped protrusion and/or the spiral anchor protrusion when the anchor is in the passive configuration. In this way, the protrusion and/or the anchor can be at least partially concealed when the device body is guided through the vaginal canal and the at least partially dilated cervix.

The needle-shaped protrusion comprising at least part of the biosensor may protrude from a substantially central point of the substantially flat surface area of the device body. The spiral protrusion comprising at least part of the ECG and optionally part of the biosensor may protrude from a substantially central point of the substantially flat surface area of the device body or a location closer to the outer edge of the substantially flat surface area of the device body.

The anchor (needle-shape protrusion and/or spiral protrusion) may be configured such that when the anchor is inserted into the foetal tissue, the device body is held firmly against the foetal tissue. In this way, the anchor may provide sufficient resistance to withstand forces applied to the device during labour and delivery.

In an embodiment, the device may comprise an actuator for inserting the protrusion into the foetal tissue and/or for anchoring the device body to the foetal tissue. A person skilled in the art will appreciate that the device may comprise multiple actuators to control respective components of the device. In one embodiment, the actuator may be configured to move the anchor from the passive configuration into the active configuration. The actuator may be in the form of a push or pull mechanism, a push-pull mechanism, a switch or a torsional mechanism.

In one particular example, the needle of the BWE and/or the spiral of the EWE/BRE may be moveable from a passive configuration at least partially inside the device body to an active configuration at least partially outside the device body to anchor the device to the foetal tissue. The needle BWE may be moved from a passive configuration inside the device body to an active configuration to anchor the device to the foetal tissue. The spiral EWE/ BRE may be moved from a passive configuration inside the device body to an active configuration to anchor the device to the foetal tissue. The advantage of a passive configuration of the needle of the BWE and/or the spiral of the EWE/BRE is that these elements will be less likely to catch on the tissue of the vagina and/or cervix and cause damage to the mother's tissue during insertion of the device. If the needle and/or spiral are in a passive configuration within the body of the device, they are also less likely to be damaged themselves by catching on the tissue of the mother. Once the device has been inserted into the vagina and/or cervix, the needle of the BWE and/or the spiral of the EWE/BRE may be moved to an active configuration to allow them to become protrusions that can be inserted at least partially into the foetal tissue to form an anchor for the device.

### Analyte Detection

In an embodiment, the device is configured such that the biosensor can detect the concentration of the analyte in foetal tissue such as the extra-cellular matrix of the skin of the foetus. Additionally, or alternatively, the device may be configured such that the biosensor can detect the concentration of the analyte in foetal blood.

In one specific embodiment, the analyte to be monitored is lactate. The concentration of lactate in the foetus may be monitored in foetal tissue and/or foetal blood. In another embodiment, the analyte to be monitored is oxygen. The concentration of oxygen in the foetus may be monitored in foetal tissue and/or foetal blood.

The measured current is proportional to the concentration of the analyte in the foetal tissue. Using this correlation, it may be possible to determine an absolute value of the analyte concentration in the foetal tissue. However, it may be sufficient to determine a trend relative to a baseline concentration and/or whether an electronic signal exceeds or falls below a predetermined threshold based on historical data. In order to determine an absolute value of the concentration of analyte in the foetus, a calibration method may need to be conducted. In this regard, a blood sample of the foetus may be taken and examined to determine a reference concentration. Having regard to exemplary values of the absolute concentration of lactate in foetal tissue, a normal range may be considered to be less than 4.1 mmol/L, whereas a pre-acidotic range may be defined at a lactate concentration between 4.2 and 4.8 mmol/L and an acidotic range may be above 4.8 mmol/L. Exemplary values of the absolute concentration of analyte in foetal tissue, a normal range may be considered to be 85-95% dissolved oxygen saturation in arterial blood, while oxygen saturation of less than 85% may be considered threatening.

In other embodiments, other analytes are envisaged, including but not limited to glucose, cortisol, pyruvate, activin A, bicarbonate, hydrogen ions, non-protein bound iron, hypoxanthine, oxygen and other suitable analytes that may be indicative of a well-being of the foetus.

In a specific embodiment, the device comprises a plurality of biosensors to monitor the concentration of a plurality of analytes.

In an embodiment, the reactive substance of the biosensor comprises an immobilised enzyme. The biosensor may be coated with the immobilised enzyme. If the device is configured to monitor a concentration of lactate of the foetus, the immobilised enzyme may, for example, be lactate oxidase or lactate dehydrogenase. However, other suitable enzymes are envisaged. A person skilled in the art will appreciate that the substance may be selected depending on the analyte to be monitored for the electrochemical reaction to occur.

If the analyte to be detected is oxygen, preferably the reactive substance of the biosensor is platinum or other noble metals. The reaction of oxygen with platinum metal in an electrochemical cell is well known, and forms the basis of a Clark electrode. The use of platinum in this application allows for the reduction of dissolved oxygen gas. Other noble metals that may be used include ruthenium, rhodium, palladium, osmium, iridium, gold or silver.

### Analysis Component

The device may further comprise an analysis component for analysing the detected electronic signal(s). The analysis component may be part of a computing device comprising a processor. The processor may be configured to determine an absolute concentration of the analyte in the foetus. In one embodiment, the analysis component is configured to determine whether the concentration of the analyte exceeds or falls below a predetermined threshold.

The analysis component may be housed in an external housing and in electronic communication with the biosensor and/or the further electrode for detecting the heart rate of the foetus. For example, the analysis component may be connected to the biosensor and/or the further electrode through a wire. The wire may extend through the guiding tube.

The external housing may be attachable to a body part of the mother. For example, the external housing may comprise a strap for attaching the analysis component to a leg of the mother. However, other configurations are envisaged. For example, the device may comprise a wireless transmitter for transmitting the detected electronic signal to a computing device, such as a mobile computing device.

The device may be configured to monitor the concentration of the analyte in real time. For example, the device may be configured to monitor the concentration of the analyte continuously, periodically or upon request.

### Monitoring System

In accordance with another embodiment, there is provided a system for simultaneously monitoring a concentration of an analyte in a foetus and a heart rate of the foetus, the system comprising:
A. a foetal monitoring device comprising five electrodes, the device comprising:
   a) two electrodes forming a biosensor wherein:
      (i) the first electrode is a biosensor working electrode (BWE), comprising a reactive substance, for monitoring the analyte concentration of the foetus,
      (ii) the second electrode is a biosensor reference electrode (BRE) for monitoring the analyte concentration of the foetus,
   b) three electrodes forming an electrocardiogram (ECG) wherein:
      (iii) the third electrode is an electrocardiogram working electrode (EWE),
      (iv) the fourth electrode is an electrocardiogram reference electrode (ERE) for monitoring the heart rate of the foetus,
      (v) the fifth electrode is an electrocardiogram ground electrode (EGE), connected externally to the mother, for monitoring the heart rate of the foetus
   c) a device body for supporting the five electrodes, the device body being configured to contact a surface area of the foetal tissue;
B. an analysis component in electronic communication with the biosensor and/or the ECG, the analysis component being configured to determine the concentration of the analyte and/or the foetal heart rate using the detected electronic signal of the biosensor and/or ECG

wherein the first electrode is not in physical contact with, or is electrically isolated from, the second electrode;
wherein the third electrode is not in physical contact with the first electrode;
wherein the fourth electrode is not in physical contact with the first, second, or third electrode;
wherein the fifth electrode is not in physical contact with the first, second, third, or fourth electrode;
wherein the foetal monitoring device is configured such that:
   the first, second and third electrodes form one or more protrusions that can be at least partially inserted into the foetal tissue so that (i) the analyte in the foetal tissue electrochemically reacts with the reactive substance of the first electrode (BWE) and in response to the electrochemical reaction the first electrode (BWE) of the biosensor detects an electronic signal, wherein a parameter of the electronic signal is indicative of the concentration of the analyte or a rate of change of concentration of the analyte; (ii) the third electrode (EWE) of the ECG detects the electrical activity of the foetal heart; and (iii) when the device body contacts the surface area of the foetal tissue, the device body can be anchored to the foetal tissue using the first, second, and third electrodes as an anchor to secure the device body against the contacted surface area of the foetal tissue.

Preferably the analyte is a lactate analyte or an oxygen analyte. If the analyte is a lactate analyte, preferably the reactive substance of the first electrode is an immobilised enzyme. If the analyte is an oxygen analyte, preferably the reactive substance of the first electrode is platinum or other noble metals.

In one embodiment, the analysis component is configured to determine a trend of a baseline concentration of the analyte and/or the electrocardiogram (ECG). The analysis component may be configured to determine whether the concentration of the analyte and/or the ECG exceeds or falls below a predetermined threshold.

In one embodiment, the analysis component may be configured to determine an absolute concentration of the analyte in the foetus.

The system may comprise a computing device comprising the analysis component, for example, in the form of a processor. The computing device may further comprise a display for displaying information indicative of the concentration of the analyte and/or the ECG in the foetus. The information may be displayed in real-time.

The system may comprise an external housing for housing the analysis component. The external housing may comprise an attachment for attaching the external housing to a body part of the mother. For example, the external housing may comprise a strap for attaching the external housing to a leg or belly of the mother.

The analysis component may also be in electronic communication with the foetal electrode and/or the maternal electrode. The analysis component may be connected to the biosensor or the ECG apparatus through a wire. The wire may extend through a guiding tube of the device. Alternatively, the device may comprise a wireless transmitter for transmitting the detected electronic signal to a computing device, such as a mobile computing device.

### Method for Monitoring

In accordance with another embodiment, there is provided a method of monitoring a concentration of an analyte in a foetus, the method comprising the steps of:
I. providing a device for monitoring a concentration of an analyte in a foetus, the device comprising:
   a) two electrodes forming a biosensor for monitoring a concentration of an analyte in the foetus wherein:
      (i) the first electrode is a biosensor working electrode (BWE), comprising a reactive substance, for monitoring the analyte concentration of the foetus,
      (ii) the second electrode is a biosensor reference electrode (BRE) for monitoring the analyte concentration of the foetus,
   b) three electrodes forming an electrocardiogram (ECG) for monitoring the heart rate of the foetus wherein:
      (iii) the third electrode is an electrocardiogram working electrode (EWE),
      (iv) the fourth electrode is an electrocardiogram reference electrode (ERE) for monitoring the heart rate of the foetus,
      (v) the fifth electrode is an electrocardiogram ground electrode (EGE), connected externally to the mother, for monitoring the heart rate of the foetus,
   c) a device body for supporting the five electrodes, the device body being configured to contact a surface area of foetal tissue

   wherein the first electrode is not in physical contact with, or is electrically isolated from, the second electrode;
   wherein the third electrode is not in physical contact with the first electrode;
   wherein the fourth electrode is not in physical contact with the first, second, or third electrode;
   wherein the fifth electrode is not in physical contact with the first, second, third, or fourth electrode;
   wherein the foetal monitoring device is configured such that:
      the first, second and third electrodes form one or more protrusions that can be at least partially inserted into the foetal tissue so that (i) the analyte in the foetal tissue electrochemically reacts with the reactive substance of the first electrode (BWE) and in response to the electrochemical reaction the first electrode (BWE) of the biosensor detects an electronic signal, wherein a parameter of the electronic signal is indicative of the concentration of the analyte or a rate of change of concentration of the analyte; (ii) the third electrode (EWE) of the ECG detects the electrical activity of the foetal heart; and (iii) when the device body contacts the surface area of the foetal tissue, the device body can be anchored to the foetal tissue using the first, second, and third electrodes as an anchor to secure the device body against the contacted surface area of the foetal tissue.
II. inserting the protrusion at least partially into foetal tissue wherein when the protrusion has been at least partially inserted into the foetal tissue it anchors the device to the foetal tissue; and
III. detecting an electronic signal at the electrode of the biosensor, the electronic signal being in response to an electrochemical reaction between the reactive substance of the biosensor and the analyte to be monitored and/or the electronic signal being in response to the electrical activity of the foetal heart as measured by the ECG apparatus;
wherein the method is conducted such that the detected electronic signal is indicative of the concentration of the analyte in the foetus and/or the heart rate of the foetus.

Preferably the analyte is a lactate analyte or an oxygen analyte. If the analyte is a lactate analyte, preferably the reactive substance of the first electrode is an immobilised enzyme. If the analyte is an oxygen analyte, preferably the reactive substance of the first electrode is platinum or other noble metals.

In one embodiment, the protrusion is at least partially inserted into the foetal tissue such that the reactive substance can react with the analyte in the foetal tissue and/or foetal blood.

The method may comprise a step of guiding the device through the vaginal canal and dilated cervix. Specifically, the method may comprise a step of providing a guiding element comprising a base tube and a sleeve and positioning the device in a space formed between the base tube and the sleeve. When the protrusion has been inserted into the foetal tissue and the device body has been anchored to the foetal tissue, the method may comprise a step of removing the guiding element from the device.

### General

Those skilled in the art will appreciate that the invention described herein is susceptible to variations and modifications other than those specifically described. The invention includes all such variation and modifications. The invention also includes all of the steps, features, formulations and compounds referred to or indicated in the specification, individually or collectively and any and all combinations or any two or more of the steps or features.

The present invention is limited by the scope of the claims.

The invention described herein may include one or more range of values (eg. Size, displacement and field strength etc). A range of values will be understood to include all values within the range, including the values defining the range, and values adjacent to the range which lead to the same or substantially the same outcome as the values immediately adjacent to that value which defines the boundary to the range. Accordingly, unless indicated to the contrary, the numerical parameters set forth in the specification and claims are approximations that may vary depending upon the desired properties sought to be obtained by the present invention. Hence "about 80 %" means "about 80 %" and also "80 %". At the very least, each numerical parameter should be construed in light of the number of significant digits and ordinary rounding approaches.

Throughout this specification, unless the context requires otherwise, the word "comprise" or variations such as "comprises" or "comprising", will be understood to imply the inclusion of a stated integer or group of integers but not the exclusion of any other integer or group of integers. It is also noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs. The term "active agent" may mean one active agent, or may encompass two or more active agents.

The following examples serve to more fully describe the manner of using the above-described invention, as well as to set forth the best modes contemplated for carrying out various aspects of the invention. It is understood that these methods in no way serve to limit the true scope of this invention, but rather are presented for illustrative purposes.

### EXAMPLES

Further features of the present invention are more fully described in the following non-limiting Examples. This description is included solely for the purposes of exemplifying the present invention. It should not be understood as a restriction on the broad description of the invention as set out above.

In the Examples, the following abbreviations will be used:
- biosensor working electrode (BWE) - first electrode
- biosensor reference electrode (BRE) - second electrode
- ECG working electrode (EWE) - third electrode
- ECG reference electrode (ERE) - fourth electrode
- ECG ground electrode (EGE) - fifth electrode
- biosensor counter electrode (BCE)

### Example 1

### Examples of Foetal Monitoring Devices

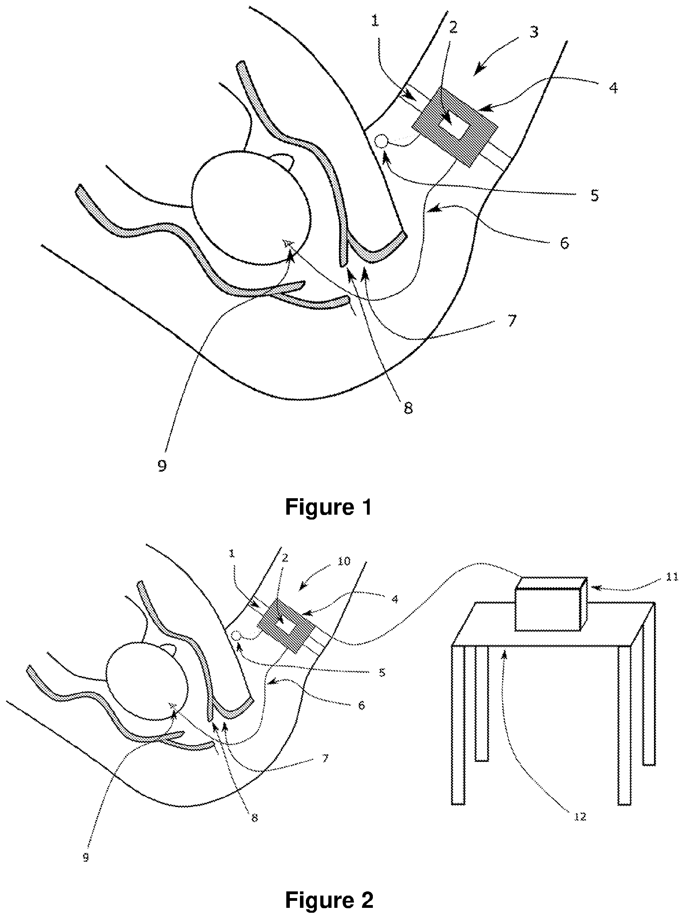

Referring now to the drawings, Figure 1 shows a system 3 for monitoring a concentration of an analyte in a foetus and a heart rate of the foetus. The system 3 comprises a device 9 to be secured to foetal tissue, in this example to a foetal scalp. A person skilled in the art will appreciate that the device 9 may be attachable to a different body part of the foetus, for example, if the foetus is in breech position. The device 9 is in electronic communication with an analysis component 4 via a wire 6. The analysis component 4 is typically a computing device comprising a processor that is, in this example, housed in an external housing 4 with a display 2 to display information indicative of the concentration of lactate and the heart rate. The external housing 4 has a strap 1 for attaching the analysis component 4 to the mother. In this example, the analysis component 4 is attached to a leg of the mother. However, the analysis component 4 may alternatively be attached to the belly of the mother or any other suitable body part. Attaching the analysis component 4 to the leg of the mother has the advantage that the length of the wire 6 can be kept relatively short and allows the mother to move around freely, walk and shower. The analysis component 4 is also attached to an external fifth electrode 5. In this example, the fifth electrode 5 is attached to the mother's leg but a person skilled in the art will appreciate this can be incorporated into analysis component 4 or attached to any other suitable body part.

The device 9 comprises at least a biosensor for sensing a concentration of the analyte, in this particular example, a concentration of lactate. The concentration of lactate is typically sensed in the foetal tissue but may also be sensed in foetal blood.

The device 9 is positioned at the foetal scalp via the vaginal canal 7 and a sufficiently dilated cervix 8. This may be done by attaching the device 9 to a guiding element such as an insertion tube. If the device 9 has a width between 0.3 and 4 cm, the device 9 can be positioned at the foetal scalp at an early phase of maternal dilation ranging from 2 to 4 cm.

An alternative exemplary system 10 in accordance with an embodiment of the present invention is illustrated in Figure 2. In this example, numerals shared with Figure 1 refer to like components of the system 10. The system 10 comprises the device 9 attachable to the foetal scalp. In this example, the device 9 is connected by wire 6 to external monitoring device 4. External monitoring device 4 may alternatively be connected wirelessly to device 9. In addition, external monitoring device 4 may be connected by a wire to computing device 11 that may be positioned on a bedside table 12. The computing device 11 may be a smartphone, tablet, laptop or personal computer. The external monitoring device 4 may alternatively be connected wirelessly to the computing device 11. In such case, the external monitoring device 4 may comprise a transmitter for wirelessly transmitting an electronic signal to the computing device 11.

The external monitoring device 4 may comprise a display 2 for displaying information indicative of the concentration of the analyte and the heart rate. The computing device 11 may also or alternatively comprises a display 33 for displaying information indicative of the concentration of the analyte and the heart rate.

The information of display 2 or display 33 may be in any suitable form. For example, the absolute concentration of the analyte may be displayed using numbers or a graph. Additionally, or alternatively, a trend from a baseline concentration may be displayed. The heart rate may be provided as a graph of voltage versus time of the electrical activity of the heart. In this way, a doctor or midwife is able to monitor the concentration of the analyte and the heart rate continuously and in real-time. Thus, if the information indicative of the concentration of lactate exceeds or falls below a predetermined threshold or the heart rate changes, the doctor or midwife are able to intervene immediately.

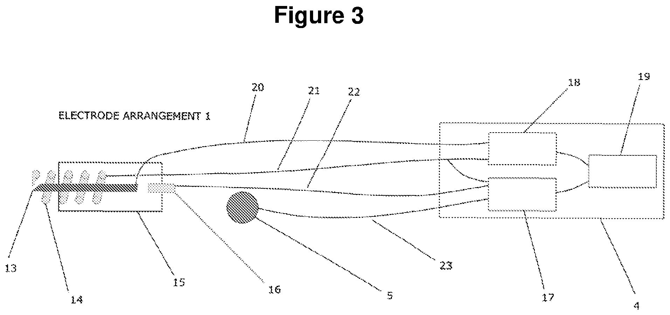

Figure 3 shows a device 9 for monitoring a concentration of an analyte and a foetal heart rate in accordance with the present invention. In this example, the device 9 is configured to monitor a concentration of lactate and the heart rate of a foetus. The device 9 may be part of system 3 or system 10 shown in Figures 1 and 2.

The first electrode 13 forms the biosensor working electrode (BWE) of the biosensor. The BRE/EWE electrode 14 forms the biosensor reference electrode (BRE) second electrode of the biosensor and the ECG working electrode (EWE) third electrode of the ECG apparatus. The fourth electrode 16 forms the ECG reference electrode (ERE) of the ECG apparatus. The fifth electrode 5 forms the ECG ground electrode (EGE) of the ECG apparatus.

The BRE/EWE electrode 14 is a separate component from the first electrode 13. Specifically, the BRE/EWE electrode 14 is in the form of a spiral that can be inserted into the foetal tissue by rotating the device body 15.

The combination of first electrode 13 and BRE/EWE electrode 14, once inserted into the foetal tissue, anchor the device body 15 to the surface area of the biological tissue.

The device 9 further comprises a fourth electrode 16. The fourth electrode 16 is a separate component from the first electrode 13 and the BRE/EWE electrode 14. The fourth electrode 16 is located within the body of the device body 15.

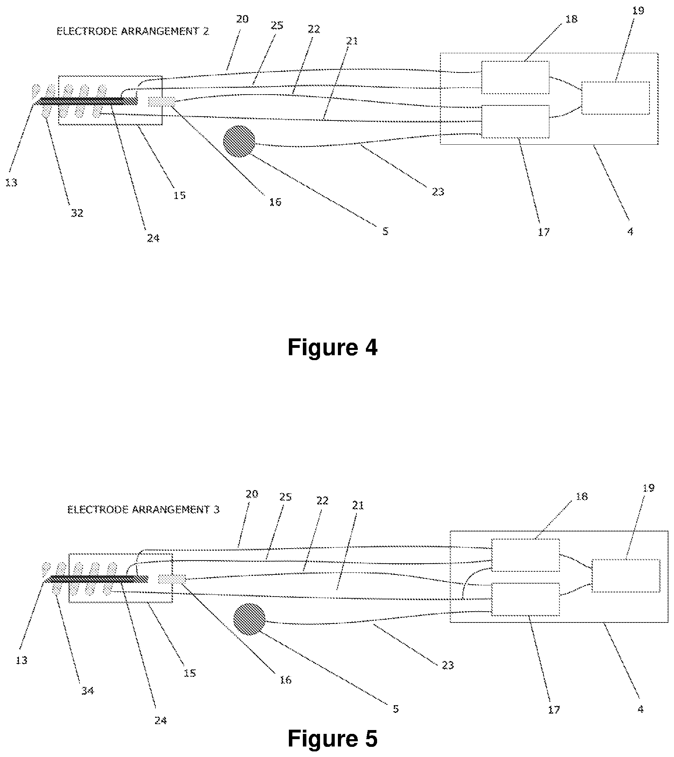

Figure 4 shows an alternative (unclaimed) embodiment of device 9 for monitoring a concentration of an analyte and a foetal heart rate in accordance with the present invention.

The first electrode 13 forms the biosensor working electrode (BWE) of the biosensor. The second electrode 24 forms the biosensor reference electrode (BRE) of the biosensor. The third electrode 32 forms the ECG working electrode (EWE) of the ECG apparatus. The fourth electrode 16 forms the ECG reference electrode (ERE) of the ECG apparatus. The fifth electrode 5 forms the ECG ground electrode (EGE) of the ECG apparatus.

The second electrode 24 is electrically insulated from the first electrode 13. Specifically, the second electrode 24 is in the form of a micro-coil or metal layer that surrounds the upper portion of first electrode 13 to a distance of approximately 1 mm, 2 mm, 3 mm, 4 mm, 5 mm, 10 mm, 20 mm, 30, mm, 40 mm or 50 mm.

The third electrode 32 is physically isolated from the first electrode 13 and second electrode 24. Specifically, the third electrode 32 is in the form of a spiral that can be inserted into the foetal tissue by rotating the device body 15. The combination of first electrode 13 and third electrode 32, once inserted into the foetal tissue, anchor the device body 15 to the surface area of the biological tissue.

The device 9 further comprises a fourth electrode 16. The fourth electrode 16 is a separate component from the first electrode 13, second electrode 24, and the third electrode 14. The fourth electrode 16 is located within the body of the device body 15.

The device 9 further comprises fifth electrode 5 that forms the ECG ground electrode (EGE) of the ECG apparatus.

The combination of third electrode 32, fourth electrode 16 and fifth electrode 5 comprise the ECG apparatus.

Figure 5 shows an alternative (unclaimed) embodiment of device 9 for monitoring a concentration of an analyte and a foetal heart rate in accordance with the present invention.

The first electrode 13 forms the biosensor working electrode (BWE) of the biosensor. The second electrode 24 forms the biosensor reference electrode (BRE) of the biosensor. The EWE/BCE electrode 34 forms the ECG working electrode (EWE) of the ECG apparatus and the biosensor counter electrode (BCE) of the biosensor apparatus. The fourth electrode 16 forms the ECG reference electrode (ERE) of the ECG apparatus. The fifth electrode 5 forms the ECG ground electrode (EGE) of the ECG apparatus.

The second electrode 24 is electrically insulated from the first electrode 13. Specifically, the second electrode 24 is in the form of a micro-coil or metal layer that surrounds the upper portion of first electrode 13 to a distance of approximately 1 mm, 2 mm, 3 mm, 4 mm, 5 mm, 10 mm, 20 mm, 30, mm, 40 mm or 50 mm.

The EWE/BCE electrode 34 is physically isolated from the first electrode 13 and second electrode 24. Specially, the EWE/BCE electrode 34 is in the form of a spiral that can be inserted into the foetal tissue by rotating the device body 15. The combination of first electrode 13 and EWE/BCE electrode 34, once inserted into the foetal tissue, anchor the device body 15 to the surface area of the biological tissue.

The device 9 further comprises a fourth electrode 16. The fourth electrode 16 is a separate component from the first electrode 13, second electrode 24, and the EWE/BCE electrode 34. The fourth electrode 16 is located within the body of the device body 15.

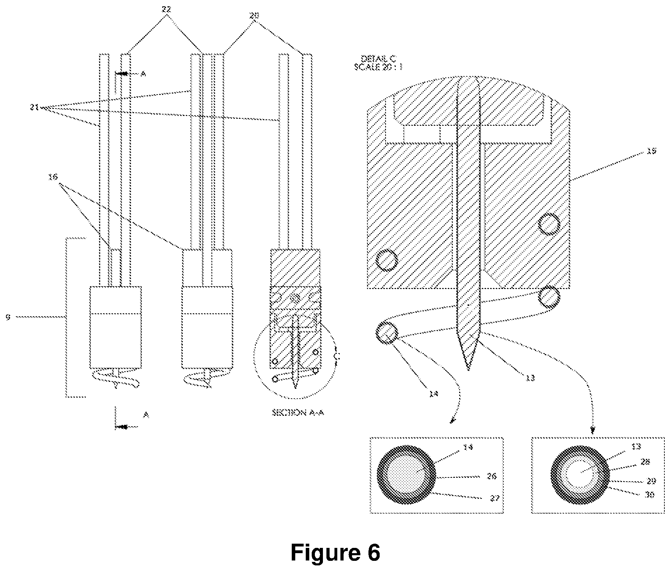

A person skilled in the art will appreciate that the device body 15 may have any suitable shape. For example, the device body 15 may have a substantially cylindrical shape, wherein the protrusion and the anchor are positioned at a proximal end of the device body, as shown in the embodiments in Figures 3 and 6.

A more detailed representation of a biosensor and ECG apparatus, wherein the BRE/EWE electrode 14 forms both the biosensor reference electrode (BRE) second electrode of the biosensor and the ECG working electrode (EWE) third electrode of the ECG apparatus is illustrated in Figure 6. In this example, the biosensor utilises a two-electrode system comprising a first electrode 13 and BRE/EWE electrode 14.

The first electrode 13, which may be made of any conductive material such as metal (e.g. platinum, stainless steel), is first coated with mediator layer 28 and then coated in reactive substance 29, has an end that forms a needle point to penetrate foetal tissue. The reactive substance 29 may be, for example, an immobilised enzyme, such lactate oxidase or lactate dehydrogenase.

The BRE/EWE electrode 14 may be a spiral made of any conductive material such as metal (e.g. platinum, stainless steel). It is then coated with Ag/AgCl or saturated calomel reference layer 26 which provides a stable and reproducible potential to which the working electrode potential is compared. The BRE/EWE electrode 14 may further comprise a membrane layer 27. The membrane layer 27 may comprise polyethylene, polyurethane or polypropylene, which acts to enhance the mechanical stability of the reference layer 26.

The biosensor is configured to measures a current between the first electrode 13 and the BRE/EWE electrode 14 and utilises the principle that the measured current is proportional to a concentration of the lactate in the foetal tissue. When the end of the first electrode 13, coated with the immobilised enzyme 29 is inserted into the foetal tissue, the immobilised enzyme 29 reacts with the lactate present in the extra-cellular matrix of the skin and blood of the foetus. The enzymatic reaction leads to the formation of peroxide ions, which are electrochemically detected as chemical reduction events at the first electrode 13 of the biosensor. This process is eased by use of mediator layer 28, which allows for a lower applied potential to electrochemically detect peroxide ions. The mediator layer may be, for example, Prussian blue. In addition, the surface of the first electrode is preferably coated with an additional layer outside the mediator layer forming a protective layer 30, to reduce the mass transport of undesirable ions to the surface of the electrode. This protective layer 30 may be, for example, a sulfonated tetrafluoroethylene-based fluoropolymer-copolymer (e.g. Nafion^{™}) or tetrafluoroethylene-based polymer (e.g. Teflon^{™}).

When the end of spiral-shaped BRE/EWE electrode 14 is inserted into the foetal tissue, an electrical circuit is completed through the third electrode 32 and the ECG reference electrode (ERE) fourth electrode 16, allowing the electrical impulse of the foetal heart to be detected as an electrical signal as the potential difference between the BRE/EWE electrode 14 and fourth electrode 16 is assessed.

An electronic signal is transmitted to an analysis component, such as ECG component 17 as shown in Figures 3-5. The electronic signal may undergo signal transformation to make available information indicative of the heart rate of the foetus. Further, once the ECG ground electrode (EGE) fifth electrode 5 as shown in Figures 3-5 has been placed into position (for example on the mother's leg) the fifth electrode 5 will allow background electrical noise to be filtered. This results in an improvement in the electrical signal that is transmitted to an analysis component, for example ECG component 17 as shown in Figures 3-5.

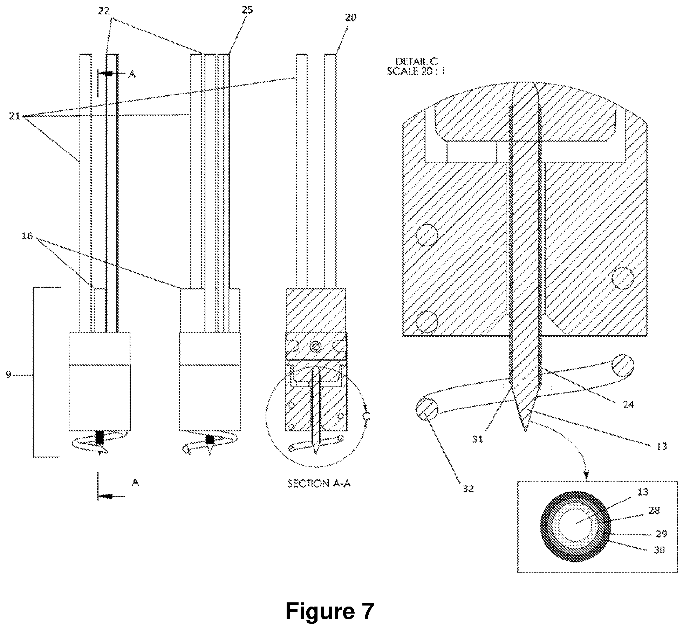

A more detailed representation of a biosensor and ECG apparatus wherein the BRE second electrode 24 and EWE third electrode 32 are separate is illustrated in Figure 7. In this (unclaimed) example, the biosensor utilises a two-electrode system comprising a first electrode 13 and a second electrode 24.

The first electrode 13, which may be made of any conductive material such as metal (e.g. platinum, stainless steel), is first coated with mediator layer 28 and then coated in reactive substance 29, has an end that forms a needle point to penetrate foetal tissue. The reactive substance 29 may be, for example, an immobilised enzyme, such lactate oxidase or lactate dehydrogenase. The BRE second electrode 24 may be an Ag/AgCl or saturated calomel electrode which provides a stable and reproducible potential to which the potential of the first electrode 13 is compared. The second electrode 24 remains electrically insulated from the first electrode 13 by insulating layer 31. The insulating layer 31 may take the form of a dielectric ink.

The biosensor is configured to measure a current between the first electrode 13 and the second electrode 24 and utilises the principle that the measured current is proportional to a concentration of the lactate in the foetal tissue. When the end of the first electrode 13, coated with reactive substance 29 is inserted into the foetal tissue, the reactive substance 29 reacts with the lactate concentrations present in the extra-cellular matrix of the skin and blood of the foetus. The enzymatic reaction leads to the formation of peroxide ions, which are electrochemically detected as chemical reduction events at the first electrode 13 of the biosensor. This process is eased by use of mediator layer 28, which allows for a lower applied potential to electrochemically detect peroxide ions. The mediator layer may be, for example, Prussian blue. In addition, the surface of the first electrode is coated with a protective layer 30, to reduce the mass transport of ions to the surface of the electrode. This protective layer 30 may be, for example, a sulfonated tetrafluoroethylene-based fluoropolymer-copolymer (e.g. Nafion^{™}) or tetrafluoroethylene-based polymer (e.g. Teflon^{™}).

An electronic signal is transmitted to an analysis component, such as biosensor component 18 as shown in Figures 3-5. The electronic signal may undergo signal transformation to make available information indicative of the concentration of the lactate in the foetus.

When the end of third electrode 32 is inserted into the foetal tissue, an electrical circuit is completed through the third electrode 32 and the ECG reference electrode (ERE) fourth electrode 16, allowing the electrical impulse of the foetal heart to be detected as an electrical signal as the potential difference between the third electrode 32 and fourth electrode 16 is assessed. This results in the transmission of an electrical signal to an analysis component, such as ECG component 17 as shown in Figures 3-5.

The electronic signal may undergo signal transformation to make available information indicative of the heart rate of the foetus. Further, once the ECG ground electrode (EGE) fifth electrode 5 as shown in Figures 3-5 has been placed into position (for example on the mother's leg) the fifth electrode 5 will allow background electrical noise to be filtered. This results in an improvement in the electrical signal that is transmitted to an analysis component, for example ECG component 17 as shown in Figures 3-5.

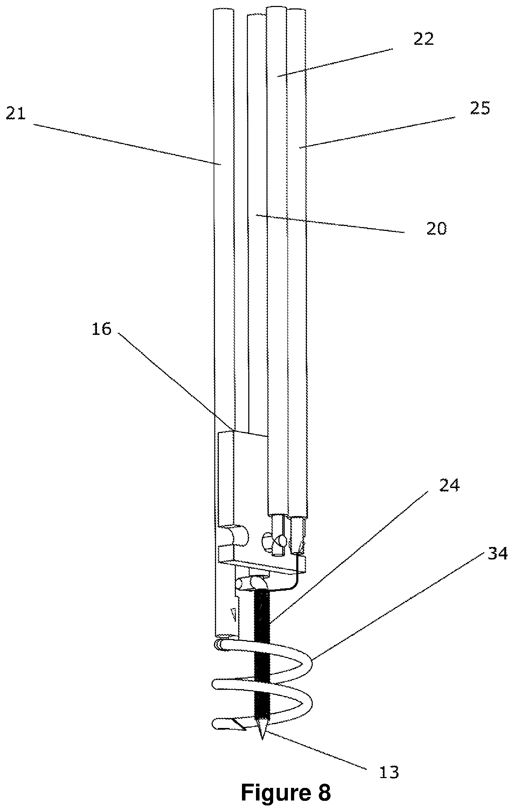

A more detailed representation of a biosensor and ECG apparatus wherein the BRE second electrode 24 and EWE/BCE electrode 34 are separate is illustrated in Figure 8. In this (unclaimed) example, the biosensor utilises a three-electrode system comprising a first electrode 13, a second electrode 24, and a EWE/BCE electrode 34.

The first electrode 13, which is coated in a reactive substance 29, has an end that forms a needle point to penetrate foetal tissue. The reactive substance 29 may be, for example, an immobilised enzyme, such lactate oxidase or lactate dehydrogenase. The second electrode 24 may be an Ag/AgCl or saturated calomel electrode which provides a stable and reproducible potential to which the potential of the first electrode 13 is compared. The second electrode 24 remains electrically insulated from the first electrode 13 by insulating layer 31, which may take the form of dielectric ink. The EWE/BCE electrode 34 may be any substantially electrically conductive material.

The biosensor is configured to measures a current between the first electrode 13 and the EWE/BCE electrode 34 at a voltage compared to second electrode 24 and utilises the principle that the measured current is proportional to a concentration of the lactate in the foetal tissue. When the end of the first electrode 13, coated with the immobilised enzyme 29 is inserted into the foetal tissue, the reactive substance 29 reacts with the lactate concentrations present in the extra-cellular matrix of the skin and blood of the foetus. The enzymatic reaction leads to the formation of peroxide ions, which are electrochemically detected as chemical reduction events at the first electrode 13 of the biosensor. This process is eased by use of a mediator layer 28, which allows for a lower applied potential to electrochemically detect peroxide ions. The mediator layer 28 may be, for example Prussian blue. In addition, the surface of the first electrode 13 is coated with a protective layer 30, to reduce the mass transport of ions to the surface of the electrode. This protective layer 30 may be, for example, a sulfonated tetrafluoroethylene-based fluoropolymer-copolymer (e.g. Nafion^{™}) or tetrafluoroethylene-based polymer (e.g. Teflon^{™}).

When the first end of first electrode 13, the first end of second electrode 24 and the first end of EWE/BCE electrode 34 are inserted into the foetal tissue, an electrical circuit is completed through the amniotic fluids of the birth canal, which allows the transmission of an electrical signal to an analysis component, such as biosensor component 18 as shown in Figures 3-5.

When the end of EWE/BCE electrode 34 is inserted into the foetal tissue, an electrical circuit is completed through the third electrode 32 and the ECG reference electrode (ERE) fourth electrode 16, allowing the electrical impulse of the foetal heart to be detected as an electrical signal as the potential difference between the EWE/BCE electrode 34 and fourth electrode 16. This results in the transmission of an electrical signal to an analysis component, such as ECG component 17 as shown in Figures 3-5. The electronic signal may undergo signal transformation to make available information indicative of the heart rate of the foetus. Further, once the ECG ground electrode (EGE) fifth electrode 5 as shown in Figures 3-5 has been placed into position (for example on the mother's leg) the fifth electrode 5 will allow background electrical noise to be filtered. This results in an improvement in the electrical signal that is transmitted to an analysis component, for example ECG component 17 as shown in Figures 3-5.

In this representation, the electrical connection of fourth electrode 16 to the ECG processor 17 is shown as 22. The connection of the second electrode 24 to the biosensor processor 18 is shown as 25. The connection of the first electrode 13 to the biosensor processor 18 is shown as 20. The connection of the EWE/BCE electrode 34 to the ECG processor 17 is shown as 21.

The devices of the present invention may also comprise an insertion tube and a guiding tube (not shown) that may be used to insert and guide the device 9 into the foetal tissue. The device 9 will be placed through the insertion tube, which will lock into fourth electrode 16. This will enable the physician to apply both force towards the foetal tissue and rotational force to the device body 15. The insertion tube will be placed inside the guiding tube, where the guiding tube is longer than the insertion tube. The physician will then insert the loaded guiding tube into the cervical canal, the guiding tube protecting the cervical canal from lacerations by the first electrode 13, BRE/EWE electrode 14, third electrode 32 or EWE/BCE electrode 34. The physician will then push the insertion tube forward and twist, inserting device 9 into the foetal tissue. The insertion tube and guiding tube can then be pulled out, leaving device body 15 inserted into the foetal tissue. This system is referred to in US Patent No: 3,827,428.

The devices of the present invention may be used in combination with a Doppler ultrasound device which is routinely used to monitor progress of labour and maternal wellbeing.

### Example 2

### Use of Foetal Monitoring Devices

### Experimental Procedure

Foetal sheep trials are regarded as the most robust proxy environment in which to study intrapartum physiology, in an environment which closely resembles the human birth environment.

Term Merino sheep (140-145 days pregnant) were used for these experiments. The lamb was partially exteriorised via a cesarean section. The exposed foetal lamb scalp was connected to several foetal monitoring devices, with intravenous (IV) and intra arterial (IA) cannulas inserted into the forelimbs of the foetal lamb. These two cannulas allowed for the simultaneous monitoring of blood pressure, temperature, blood lactate, and partial oxygen pressure (pO₂). A blood gas machine Siemens RAPIDPoint 500 was used to collect the values of lactate and PO₂. A silicone occluder is placed around the umbilical cord; the occluder is inflated with saline to occlude the umbilicus. This prevents the passage of oxygenated blood from the ewe to the lamb, simulating a hypoxic event in the foetal lamb.

Food, but not water was withdrawn 12 hours before surgery. Acepromazine (0.03 - 0.05 mg/kg; intramuscular) were administered as premedication 30 minutes prior to intubation. To facilitate intubation, thiopentone (10 - 15 mg/kg; IV) was administered. After intubation, anaesthesia was maintained with 2 - 3% isoflurane in oxygen. The depth of anaesthesia, maternal heart rate, respiration and fluid balance was monitored during the surgical procedure.

Blood was drawn three times over ten minutes from the foetal lamb to establish the basal lactate levels. The blood of the lamb was drawn periodically throughout this procedure, measuring blood lactate and pO₂. The foetal monitoring devices were active throughout the procedure. It was found that hypoxia could be gradually induced by occluding the umbilical cord in 5 minute cycles which included 2 minutes of cord occlusion time. Three of these occlusion cycles were formed over 110 mins.

### Results

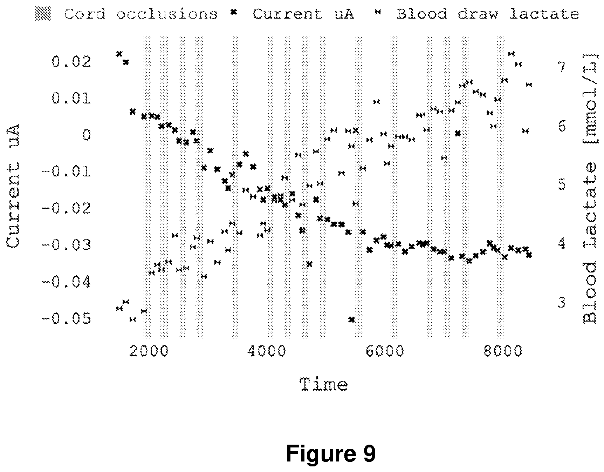

A foetal monitoring device consistent with embodiment described in Figure 3 was tested using the above experimental conditions. During the pre-occlusion, baseline measurements, the basal blood lactate was between 2.7 - 2.9 mmol/L. The umbilical cord was then occluded for 2 mins, before reversing the occlusion for 3 mins. As seen in Figure 9 upon occlusion of the umbilical cord, the blood lactate levels increased from 2.8 mmol/L to 7.2 mmol/L, consistent with a hypoxic event. The foetal monitoring device measured a decrease in current from 21 µA to -32 µA, which is inversely correlated to the rise in blood lactate (Spearman coefficient of -0.92 and p<0.05).

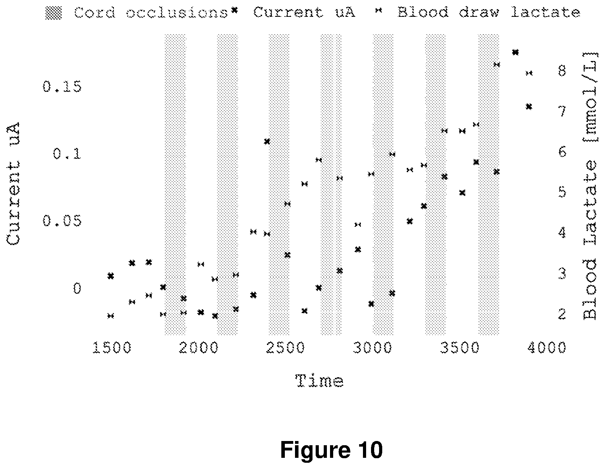

A foetal monitoring device consistent with embodiment described in Figure 4 was tested using the above experimental conditions. During the pre-occlusion, baseline measurements, the basal blood lactate was between 1.5 - 1.9 mmol/L. The umbilical cord was then occluded for 2 mins, before reversing the occlusion for 3 mins. As seen in Figure 10, upon occlusion of the umbilical cord, this increased the blood lactate level from 1.9 to 8.4 mmol/L, consistent with a hypoxic event. The foetal monitoring device measured an increase from -25 µA to 175 µA, which is positively correlated to the rise in blood lactate (Spearman coefficient of 0.85 and p<0.05).

Figure 9 shows the values of current (crosses) and venous blood lactate (double triangle). The grey areas show when cord occlusion took place. Figure 9 shows the data associated with an embodiment consistent with Figure 3. The left axis shows the scale for current (µA) and the right axis shows the blood lactate (mmol/L). The x-axis shows time measured in seconds. The effect of the occlusion on venous blood lactate can clearly be seen as it rises gradually. The inverse can be observed for current, which decreases proportionally as blood lactate rises. This suggests that the current value obtained from the foetal monitoring device may be used to monitor blood lactate in a newly born animal, allowing for continuous foetal monitoring.

Figure 10 shows the values of current (crosses) and venous blood lactate (double triangle). The grey areas show when cord occlusion took place. Figure 10 shows the data associated with an embodiment consistent with Figure 4. The left axis shows the scale for current (µA) and the right axis shows the blood lactate (mmol/L). The x-axis shows time measured in seconds. The effect of the occlusion on venous blood lactate can clearly be seen as it rises gradually. The same can be observed for current, which increases proportionally as blood lactate rises. This suggests that the current value obtained from the foetal monitoring device may be used to monitor blood lactate in a newly born animal, allowing for continuous foetal monitoring.

Therefore, it can be shown that the present invention, using embodiments shown in Figure 3 and 4, provide a means for continuous foetal monitoring of blood lactate, which may be used for simultaneously monitoring an analyte in a foetus.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive.

## Claims

1. A foetal monitoring device (9) comprising five electrodes configured to simultaneously monitor a concentration of an analyte in a foetus and a heart rate of the foetus, the device (9) comprising:
a) two electrodes forming a biosensor configured to monitor a concentration of an analyte in the foetus wherein:
(i) the first electrode (13) is a biosensor working electrode (BWE) configured to detect an electronic signal, comprising a reactive substance, configured to monitor the analyte concentration of the foetus, wherein the first electrode (13) is configured to detect an electronic signal in response to an electrochemical reaction of the first electrode (13), and wherein a parameter of the electronic signal is indicative of the concentration of the analyte or a rate of change of concentration of the analyte,
(ii) the second electrode (24) is a biosensor reference electrode (BRE) configured to monitor the analyte concentration of the foetus,
b) three electrodes forming an electrocardiogram (ECG) configured to monitor the heart rate of the foetus wherein:
(iii) the third electrode (32) is an electrocardiogram working electrode (EWE) configured to detect the electrical activity of the foetal heart,
(iv) the fourth electrode (16) is an electrocardiogram reference electrode (ERE),
(v) the fifth electrode (5) is an electrocardiogram ground electrode (EGE), configured to be connected to the mother,
c) a device body (15) for supporting the five electrodes (13, 24, 32, 16, 5), the device body (15) being configured to contact a surface area of foetal tissue
wherein the first electrode (13) is not in physical contact with, or is electrically isolated from, the second electrode (24);
wherein the third electrode (32) is not in physical contact with the first electrode (13), wherein the second electrode (24) (BRE) is also the third electrode (32) (EWE) to form a combined BRE/EWE electrode (14);
wherein the fourth electrode (16) is not in physical contact with the first, second, or third electrode (13, 24, 32);
wherein the fifth electrode (5) is not in physical contact with the first, second, third, or fourth electrode (13, 24, 32, 16);
wherein the first, second and third electrodes (13, 24, 32) form one or more protrusions configured to be at least partially inserted into the foetal tissue so that the analyte in the foetal tissue electrochemically reacts with the reactive substance of the first electrode (13) (BWE); and
wherein the first, second, and third electrodes (13, 24, 32) are configured to be used as an anchor to secure the device body (15) against the contacted surface area of the foetal tissue.

2. The foetal monitoring device (9) of claim 1 wherein the analyte is a lactate analyte or an oxygen analyte.

3. The foetal monitoring device (9) of claim 2 wherein if (i) the analyte is a lactate analyte, the reactive substance of the first electrode (13) is an immobilised enzyme; or (ii) the analyte is an oxygen analyte, the reactive substance of the first electrode (13) is platinum or other noble metals.

4. The foetal monitoring device (9) of claim 1 wherein either the second electrode (24) (BRE) or the third electrode (32) (EWE) is also a biosensor counter electrode (BCE).

5. The foetal monitoring device (9) of claim 1 wherein the combined BRE/EWE electrode (14) is a biosensor counter electrode (BCE).

6. A system configured to simultaneously monitor a concentration of an analyte in a foetus and a heart rate of the foetus, the system comprising:
A. the foetal monitoring device (9) of any preceding claim, wherein
(i) the fourth electrode (16) is configured to monitor the heart rate of the foetus, and
(ii) the fifth electrode (5) is configured to monitor the heart rate of the foetus; and
B. an analysis component in electronic communication with the biosensor and/or the ECG, the analysis component being configured to determine the concentration of the analyte and/or the foetal heart rate using the detected electronic signal of the biosensor and/or ECG.

## Patentansprüche

1. Fötusüberwachungsvorrichtung (9) mit fünf Elektroden, die so konfiguriert sind, dass sie gleichzeitig die Konzentration eines Analyten im Fötus und die Herzfrequenz des Fötus überwachen, die Vorrichtung (9) umfassend:
a) zwei Elektroden, die einen Biosensor bilden, der zur Überwachung der Konzentration eines Analyten im Fötus konfiguriert ist, wobei:
(i) die erste Elektrode (13) eine Biosensor-Arbeitselektrode (BWE) ist, die zur Erkennung eines elektronischen Signals konfiguriert ist, umfassend eine reaktive Substanz, die zur Überwachung der Analytkonzentration des Fötus konfiguriert ist, wobei die erste Elektrode (13) zur Erkennung eines elektronischen Signals als Reaktion auf eine elektrochemische Reaktion der ersten Elektrode (13) konfiguriert ist, wobei ein Parameter des elektronischen Signals die Konzentration des Analyten oder eine Änderungsrate der Konzentration des Analyten anzeigt,
(ii) die zweite Elektrode (24) eine Biosensor-Referenzelektrode (BRE) ist, die zur Überwachung der Analytkonzentration des Fötus konfiguriert ist.
b) drei Elektroden, die ein Elektrokardiogramm (EKG) bilden, das zur Überwachung der Herzfrequenz des Fötus konfiguriert ist, wobei:
(iii) die dritte Elektrode (32) eine Elektrokardiogramm-Arbeitselektrode (EWE) ist, die zur Erkennung der elektrischen Aktivität des fötalen Herzens konfiguriert ist,
(iv) die vierte Elektrode (16) eine Elektrokardiogramm-Referenzelektrode (ERE) ist,
(v) die fünfte Elektrode (5) eine Elektrokardiogramm-Erdungselektrode (EGE) ist, die so konfiguriert ist, dass sie mit der Mutter verbunden werden kann,
c) einen Vorrichtungskörper (15) zum Tragen der fünf Elektroden (13, 24, 32, 16, 5), wobei der Vorrichtungskörper (15) so konfiguriert ist, dass er eine Oberfläche des fötalen Gewebes berührt.
wobei die erste Elektrode (13) nicht in physischem Kontakt mit der zweiten Elektrode (24) steht oder von dieser elektrisch isoliert ist;
wobei die dritte Elektrode (32) nicht in physischem Kontakt mit der ersten Elektrode (13) steht, wobei die zweite Elektrode (24) (BRE) auch die dritte Elektrode (32) (EWE) ist, um eine kombinierte BRE/EWE-Elektrode (14) zu bilden;
wobei die vierte Elektrode (16) nicht in physischem Kontakt mit der ersten, zweiten oder dritten Elektrode (13, 24, 32) steht;
wobei die fünfte Elektrode (5) nicht in physischem Kontakt mit der ersten, zweiten, dritten oder vierten Elektrode (13, 24, 32, 16) steht;
wobei die erste, zweite und dritte Elektrode (13, 24, 32) einen oder mehrere Ausbuchtungen bilden, die so konfiguriert sind, dass sie mindestens teilweise in das fötale Gewebe eingeführt werden können, sodass der Analyt im fötalen Gewebe elektrochemisch mit der reaktiven Substanz der ersten Elektrode (13) (BWE) reagiert; und
wobei die erste, zweite und dritte Elektrode (13, 24, 32) so konfiguriert sind, dass sie verwendet werden, um den Vorrichtungskörper (15) an der Kontaktfläche des fötalen Gewebes zu befestigen.

2. Fötusüberwachungsvorrichtung (9) nach Anspruch 1, wobei der Analyte ein Laktat-Analyt oder ein Sauerstoff-Analyt ist.

3. Fötusüberwachungsvorrichtung (9) nach Anspruch 2, wobei, wenn (i) der Analyt ein Laktat-Analyt ist, die reaktive Substanz der ersten Elektrode (13) ein immobilisiertes Enzym ist; oder (ii) der Analyt ein Sauerstoff-Analyt ist, die reaktive Substanz der ersten Elektrode (13) Platin oder andere Edelmetalle sind.

4. Fötusüberwachungsvorrichtung (9) nach Anspruch 1, wobei entweder die zweite Elektrode (24) (BRE) oder die dritte Elektrode (32) (EWE) ebenfalls eine Biosensor-Gegenelektrode (BCE) ist.

5. Fötusüberwachungsvorrichtung (9) nach Anspruch 1, wobei die kombinierte BRE/EWE-Elektrode (14) eine Biosensor-Gegenelektrode (BCE) ist.

6. System, das so konfiguriert ist, dass es gleichzeitig die Konzentration eines Analyten in einem Fötus und die Herzfrequenz des Fötus überwacht, das System umfassend:
A. die Fötusüberwachungsvorrichtung (9) aus einem der vorstehenden Ansprüche, wobei
(i) die vierte Elektrode (16) so konfiguriert ist, dass sie die Herzfrequenz des Fötus überwacht, und
(ii) die fünfte Elektrode (5) so konfiguriert ist, dass sie die Herzfrequenz des Fötus überwacht; und
B. eine Analysekomponente, die mit dem Biosensor und/oder dem EKG elektronisch kommuniziert, wobei die Analysekomponente so konfiguriert ist, dass sie die Konzentration des Analyten und/oder die fötale Herzfrequenz anhand des verwendeten elektronischen Signals des Biosensors und/oder des EKG verwendet.

## Revendications

1. Dispositif de surveillance fœtale (9) comprenant cinq électrodes configurées pour surveiller simultanément une concentration d'un analyte dans un fœtus et une fréquence cardiaque du fœtus, le dispositif (9) comprenant :
a) deux électrodes formant un biocapteur configuré pour surveiller une concentration d'un analyte dans le fœtus, dans lequel :
(i) la première électrode (13) est une électrode de travail de biocapteur (BWE) configurée pour détecter un signal électronique, comprenant une substance réactive, configurée pour surveiller la concentration d'analyte du fœtus, dans lequel la première électrode (13) est configurée pour détecter un signal électronique en réponse à une réaction électrochimique de la première électrode (13), et dans lequel un paramètre du signal électronique est indicatif de la concentration de l'analyte ou d'un taux de variation de la concentration de l'analyte,
(ii) la deuxième électrode (24) est une électrode de référence de biocapteur (BRE) configurée pour surveiller la concentration d'analyte du fœtus,
b) trois électrodes formant un électrocardiogramme (ECG) configuré pour surveiller la fréquence cardiaque du fœtus, dans lequel :
(iii) la troisième électrode (32) est une électrode de travail d'électrocardiogramme (EWE) configurée pour détecter l'activité électrique du cœur fœtal,
(iv) la quatrième électrode (16) est une électrode de référence d'électrocardiogramme (ERE),
(v) la cinquième électrode (5) est une électrode de masse d'électrocardiogramme (EGE), configurée pour être connectée à la mère,
c) un corps de dispositif (15) pour supporter les cinq électrodes (13, 24, 32, 16, 5), le corps de dispositif (15) étant configuré pour entrer en contact avec une surface de tissu fœtal
dans lequel la première électrode (13) n'est pas en contact physique avec, ou est électriquement isolée de, la deuxième électrode (24) ;
dans lequel la troisième électrode (32) n'est pas en contact physique avec la première électrode (13), dans lequel la deuxième électrode (24) (BRE) est également la troisième électrode (32) (EWE) pour former une électrode combinée BRE/EWE (14) ;
dans lequel la quatrième électrode (16) n'est pas en contact physique avec la première, la deuxième ou la troisième électrode (13, 24, 32) ;
dans lequel la cinquième électrode (5) n'est pas en contact physique avec la première, la deuxième, la troisième ou la quatrième électrode (13, 24, 32, 16) ;
dans lequel les première, deuxième et troisième électrodes (13, 24, 32) forment une ou plusieurs protubérances configurées pour être au moins partiellement insérées dans le tissu fœtal de sorte que l'analyte dans le tissu fœtal réagit électrochimiquement avec la substance réactive de la première électrode (13) (BWE) ; et
dans lequel les première, deuxième et troisième électrodes (13, 24, 32) sont configurées pour être utilisées comme ancrage afin de fixer le corps de dispositif (15) contre la surface de contact du tissu fœtal.

2. Dispositif de surveillance fœtale (9) selon la revendication 1, dans lequel l'analyte est un analyte de lactate ou un analyte d'oxygène.

3. Dispositif de surveillance fœtale (9) selon la revendication 2, dans lequel si (i) l'analyte est un analyte de lactate, la substance réactive de la première électrode (13) est une enzyme immobilisée ; ou (ii) l'analyte est un analyte d'oxygène, la substance réactive de la première électrode (13) est du platine ou d'autres métaux nobles.

4. Dispositif de surveillance fœtale (9) selon la revendication 1 dans lequel soit la deuxième électrode (24) (BRE) soit la troisième électrode (32) (EWE) est également une contre-électrode de biocapteur (BCE).

5. Dispositif de surveillance fœtale (9) selon la revendication 1 dans lequel l'électrode combinée BRE/EWE (14) est une contre-électrode de biocapteur (BCE).

6. Système configuré pour surveiller simultanément une concentration d'un analyte dans un fœtus et une fréquence cardiaque du fœtus, le système comprenant :
A. le dispositif de surveillance fœtale (9) selon une quelconque revendication précédente, dans lequel
(i) la quatrième électrode (16) est configurée pour surveiller la fréquence cardiaque du fœtus, et
(ii) la cinquième électrode (5) est configurée pour surveiller la fréquence cardiaque du fœtus ; et
B. un composant d'analyse en communication électronique avec le biocapteur et/ou l'ECG, le composant d'analyse étant configuré pour déterminer la concentration de l'analyte et/ou la fréquence cardiaque fœtale à l'aide du signal électronique détecté du biocapteur et/ou de l'ECG.
